# EUROPEAN PATENT APPLICATION

(11) **EP 2 028 206 A1**
(43) Date of publication of application: **25.02.2009**
(21) Application number: 07114803.5
(22) Date of filing: 23.08.2007
(51) Int. Cl.: C08G 18/76, C08G 12/08, C07C 211/54, C07C 263/10, C07C 265/12

(54) **Polyaromatic polyisocyanate compositions**

(71) Applicant: HUNTSMAN INTERNATIONAL LLC, Salt Lake City, Utah 84108 (US)
(72) Inventor: The designation of the inventor has not yet been filed
(74) Representative: Swinnen, Anne-Marie

(57) **Abstract**

Production of aromatic polyamine mixtures from formaldehyde, a mono-nuclear aromatic monoamine and one or more aromatic di- or higher functionality amines and conversion of these polyamine mixtures to the corresponding polyisocyanates by phosgenation.

## Description

The present invention relates to polyaromatic polyisocyanate compositions especially beneficial for the manufacture of polyurethanes, polyisocyanurates, polyureas and related products by means of phosgenation of particular polyamine compositions produced by economically beneficial means.

Aromatic polyamines are used for various applications in the chemical industry, for example, as the curing agent of epoxy resins, as components in polyurethanes or polyureas and as raw materials of polyamides. In particular, aromatic polyamines are useful as intermediates for manufacturing the corresponding polyisocyanates by reaction with phosgene. Polyurethanes are a widely employed group of polymers, with applications as diverse as insulation foam for construction and in domestic and industrial appliances, as flexible foams in furniture and automotive seating, as semi-rigid foams in packaging, as elastomers in shoe soles and in coatings, sealants and encapsulants. The polyisocyanates themselves can be used as the binding component in many products based on, for example, various types of wood pieces or granulated rubber.

It is an object of the present invention to provide a process for the production of certain polyamine mixtures to be used as intermediates in the production of the corresponding polyisocyanates.

We have found that this object is achieved by the production of polyamine mixtures from formaldehyde, a mono-nuclear aromatic monoamine and one or more aromatic di- or higher functionality amines.

Production of polyamine mixtures with formaldehyde and two or more aromatic amines leads to a complex mixture of compounds.
In the following description, the following terms are used :-
MDA : methylene dianiline (also known as diaminodiphenylmethane) which, unless specified otherwise, also denotes the various isomeric forms.
PMDA : the complex mixture of polyaromatic polyamines, including MDA, formed by the acid-catalysed reaction of aniline with formaldehyde
TDA : toluene diamine (also know as diaminotoluene) which, unless specified otherwise, also denotes the various isomeric forms.
MTA : Methylene di-phenylene, mono-methyl, triamine (also known as triaminomethyl-diphenylmethane) which, unless specified otherwise, also denotes the various isomeric forms.
PMTA : the complex mixture of polyaromatic polyamines, including MDA and MTA, formed by the acid-catalysed reaction of aniline and TDA with formaldehyde.

Conversion of the polyamines to the corresponding polyisocyanates by phosgenation produces respectively MDI, PMDI, TDI, MTI and PMTI.

The following description based on the use of aniline and 2,4-TDA, with aniline as the main aromatic amine and 2,4-TDA as a relatively minor component, is provided for clarity but it is to be understood not to limit the scope of the invention.

Products formed when producing polyamine mixtures from formaldehyde, aniline and 2,4-TDA include the dinuclear species AA & AB, the trinuclear species AAB & ABA, the tetranuclear species AAAB & AABA etc. in various isomeric forms [where A denotes aniline and B denotes 2,4-TDA]. Since here B is a minor component, the species BB, ABB, AABB, etc would only be present at lower levels and are not considered in the following descriptions, even though their presence in the mixtures might affect the properties of the polyamines, the derived corresponding polyisocyanates or the derived reaction products of the polyisocyanates with various other products.

Thus, present in the polyamine mixture will be :-
4,4'-MDA (plus the 2,4'-MDA and 2,2'-MDA isomers - not shown) 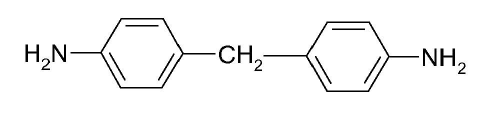 and various isomers of MTA 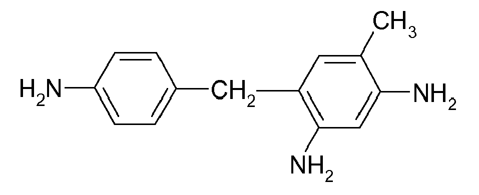 and isomers of the different types of tri-nuclear polyamines formed just from aniline or from aniline and TDA :- 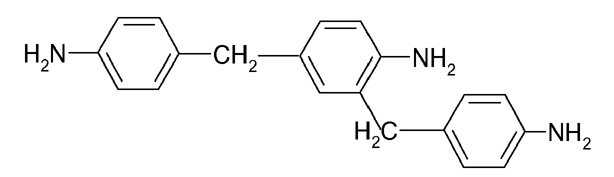 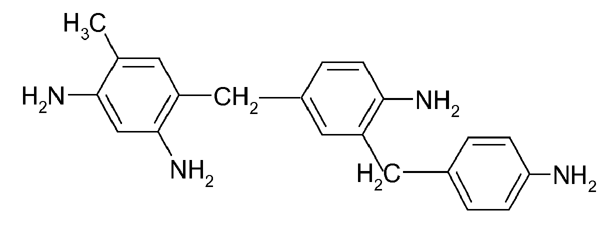 and isomers of the different types of tetra-nuclear and higher molecular weight polyamines (not shown).

It is to be understood that these structures are only representative of some of the compounds present in the polyamine mixture. There will also be higher molecular weight oligomers and various isomers of each of the different molecular weight oligomers. Phosgenation of the polyamine gives the corresponding polyisocyanates and relatively minor levels of side products produced from various reactions between species.
4,4'-MDI (plus the 2,4'-MDI and 2,2'-MDI isomers - not shown) 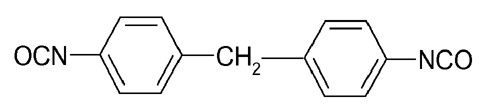 and various isomers of MTI 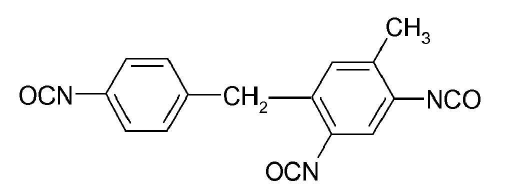 and isomers of the different types of tri-nuclear polyisocyanates 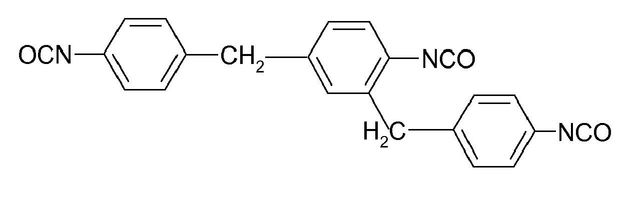 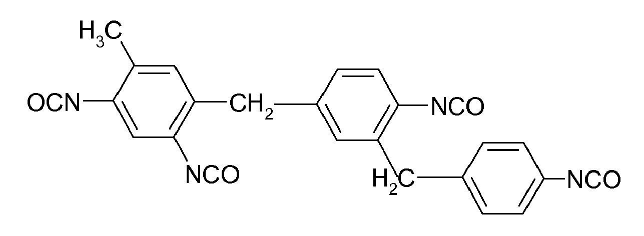 and isomers of the different types of tetra-nuclear and higher molecular weight polyisocyanates (not shown).

It is to be understood that these structures are only representative of some of the compounds present in the polyisocyanate mixture. There will also be higher molecular weight oligomers and various isomers of each of the different molecular weight oligomers and side products produced from various reactions between species.

For simplicity, the entire mixture of all the isomers of all the AA, AB, AAB, ABA, AAAB, AABA, etc polyamines is termed PMTA and the corresponding mixture of polyisocyanates is termed PMTI.

Instead of formaldehyde, materials capable of yielding the desired "CH₂O" species under the conditions of the process may also be used, for example, paraformaldehyde, trioxane, dimethoxymethane (formal) and aqueous solutions of formaldehyde (formalin) at various concentrations. Stabilisers such as methanol, polyvinylalcohol, etc. may be present in the formalin. Gaseous formaldehyde may also be employed.

Examples of mononuclear aromatic monoamines which can be used include aniline, o- and m-substituted anilines such as toluidines and alkyl anilines, chloroanilines, anisidines and nitroanilines. Preferably aniline is used.

Examples of diamines or higher functionality amines which can be used include the diaminobenzene isomers such as 1,3-phenylene diamine, alkyl-substituted diaminobenzenes such as the isomers of TDA, isomers of methylene diphenylene diamine such as 4,4'-MDA and mixtures of various mixtures of isomers and homologues of aniline-formaldehyde condensates known generally as PMDA. Particularly suitable mixtures are those containing from about 65 per cent to about 80 per cent 2,4-tolulene diamine and the balance 2,6-tolulene diamine. The commercially available mixture containing about 80 per cent 2,4- and about 20 per cent 2,6-tolulene diamine is very useful.

The used acid catalyst may be a mineral acid such as hydrochloric or sulphuric acid or a heterogeneous catalyst such as those described in Trends in industrial catalysis in the polyurethane industry, App. Catal A : General, 221, 303-335 (2001) or delaminated zeolite catalysts (WO 03/082803). Use of hydrochloric acid or gaseous hydrogen chloride in combination with aniline with an appropriate water content is preferred.

An additional benefit of the present invention is the production of polyisocyanate mixtures corresponding to the particular polyamine mixtures with improved reactivity due to the relatively low levels of so-called hydrolysable chlorine impurities.

Other additional benefits arising from various embodiments of the present invention include :-
(a) production of polyamine mixtures with low levels of nitrogen-methylated [so-called N-methyl or N-CH₃] functional groups which, ultimately, give polyisocyanate products with low levels of so-called hydrolysable chlorine impurities.
(b) economic attractiveness from the use of relatively low levels of hydrochloric acid catalyst and, hence, use of lower levels of neutralising sodium hydroxide, together with minimisation of the scale of the work-up and effluent treatment equipment and the amount of effluent.
(c) the process can be operated as a batch, continuous or semi-continuous process.
(d) the reactants can be brought together by a variety of different methods such as dip-pipes in to continuous stirred tank reactors or, preferably, by injection in to a flowing stream through a number of injection points, followed by mixing with static mixers or other mixing devices.
(e) the mono-amine and di-amine reactants may be provided in pure form or with low levels of impurities which do not affect the polyamine or polyisocyanate production or work-up processes nor have significant detrimental effects on final polyisocyanate product quality or derived polyurethane products.
(f) the formaldehyde may be provided in any suitable form, for example, as a gas, as solid paraformaldehyde or, preferably, as an aqueous solution (commonly termed formalin). The formalin may be of any suitable concentration, preferably greater that 40% formaldehyde by weight and may contain various stabilisers such as methanol and/or polyvinylalcohol. Preferably, the formalin contains low levels of di- and poly-valent metal ions to avoid formation of a rag layer after neutralisation of the acid reaction mixture.
(g) the hydrochloric acid may be provided as an aqueous solution, preferably with concentration greater that 30% by weight HCl or, optionally, it may be created in situ by absorption of gaseous hydrogen chloride, optionally derived from a phosgenation plant, either in to water or by absorption in to aniline containing an appropriate amount of water to avoid formation of aniline hydrochloride solids.
(h) solid acids may be used as catalyst.
(i) the mono-amine and di-amine reactants may be introduced to the manufacturing equipment independently, as a pre-formed mixture, with or without added hydrochloric acid or gaseous HCl or, preferably, the mono-amine may be mixed with the acid catalyst prior to introduction of the formaldehyde, the resulting reaction mixture then being reacted with the di-amine, optionally dissolved in additional mono-amine or solvent, and subsequently heated to higher temperatures to complete the rearrangement reactions. The final temperature of the reaction mixture is preferentially at least 120°C.
(j) the process for the production and work-up of the polyamine mixture and the subsequent process for the production and work-up of the polyisocyanate can be monitored and optionally controlled by means of a range of on-line analytical techniques such as near-infrared spectroscopy, UV-visible spectroscopy, gas chromatography, liquid chromatography, nuclear magnetic resonance spectrometry, mass spectrometry and the like, optionally using chemometric or other numerical or computer-based techniques for data processing.
(j) the process for the production and work-up of the polyamine mixture may be retro-fitted to existing plant designed and constructed for the production of PMDA with relatively straightforward additional engineering design and construction.

The process used in converting the polyamine mixture to the corresponding polyisocyanate mixture can be any of those described in the prior art or used commercially hitherto.

The object of the present invention in providing a process for the production of certain polyamine mixtures to be used as intermediates in the production of the corresponding polyisocyanates will now be described in terms of the reactions occurring using formaldehyde (as an aqueous solution), aniline (as the mononuclear aromatic monoamine) and the so-called "80:20" mixture of 2,4'-TDA and 2,6'-TDA (as the mononuclear aromatic diamine - hereafter simply referred to as TDA) although it is to be understood that such a description is provided for the purpose of clarity and is not limiting in any way. Likewise, descriptions of the chemical reactions or chemical species present during the reactions are also given for the benefit of clarity only and not as any distinguishing aspect of the invention.

Aniline and hydrochloric acid are mixed together and, optionally, the temperature of the resulting mixture is controlled to a prescribed level, preferably in the range 30 to 90°C. Formaldehyde is added to the aniline/hydrochloric acid mixture, optionally by means of mixing equipment, whilst maintaining the temperature below a prescribed level, preferably in the range 30 to 90°C. This mixture may be maintained at low temperature for some time or the TDA can be added within a few minutes of the end of the formaldehyde addition at a temperature preferably in the range 30 to 90°C. Alternatively, the reaction mixture formed from aniline, hydrochloric acid and formaldehyde may be allowed to continue to react at a somewhat higher temperature prior to addition of the TDA, depending upon the exact product composition required. The TDA may be added in molten form or in solution in a solvent or pre-dispersed in aniline. Some of the hydrochloric acid may also be added at this time, either separately or included with the aniline/TDA or all the hydrochloric acid can be added initially to the desired fraction of the aniline. Overall, the relative amounts of the various species brought together are :-
aniline : in the range 1.5 to 4.0 moles, preferably 1.7 to 3.5 moles per mole of formaldehyde,
HCl : in the range 0.05 to 0.60 moles, preferably 0.1 to 0.35 moles per mole of formaldehyde,
TDA : in the range 0.1 to 0.8 moles, preferably 0.2 to 0.5 moles per mole of formaldehyde.

After addition of the TDA, the reaction mixture is brought to higher temperatures for an extended time, either following a simple linear temperature ramp or as a more complex temperature/time profile in order to convert the various secondary amine species to primary amines and thus obtain the desired mixture of polyamine oligomers and their corresponding isomer ratios. The final temperature at this stage can be greater than 100°C in order to carry out the final chemical rearrangements at an economically beneficial rate.

Such temperatures require that the reaction mixture is maintained above atmospheric pressure.

Neutralisation of the acidic reaction mixture is then carried out by reaction with a suitable quantity of base. Preferably, this involves reaction with a slight stoichiometric excess of aqueous sodium hydroxide although any of the neutralisation options described in the prior art may also be applied. The neutralisation may be carried out on the polyamine mixture after cooling below the final reaction temperature or, preferably, at substantially the same temperature as the reaction in suitably designed equipment capable of withstanding the high temperatures and elevated pressures. Separation of the organic phase comprising of the predominantly aniline/TDA/polyamine mixture and the brine phase is then carried out with conventional phase separation devices. Secondary washing and subsequent separation of the phases is also preferentially carried out viz: the organic phase can be washed with additional aqueous material and the brine phase can be washed with additional aniline of suitable quality i.e. impurities may be present in the washing fluids.

Unreacted aniline and TDA are then removed from the organic mixture by any appropriate means preferentially by fractional distillation, to yield the final mixture of desired polyaromatic polyamines with amounts of the free mononuclear amines below or substantially below a total of 200 ppm by weight, preferably below 50 ppm by weight. The aniline and TDA may be recycled. To ensure very low residual TDA going forward to phosgenation, the removal of the mononuclear amines is generally accompanied by removal of some higher molecular weight compounds such as, for example, dinuclear polyamines. The recycling stream of aniline, TDA and higher molecular weight polyamines could be subjected to further treatment, for example, fractional distillation, to modify the composition before addition back to the process. However, it is preferable to use the unmodified stream containing predominantly mononuclear amines such as aniline and TDA plus the higher molecular weight polyamines and add this stream back to the production process. The presence of the higher molecular weight compounds in the recycle will have a small effect on the composition of the polyamine mixture produced but this can be adequately compensated for by changes to the ratios of the various reactants viz: aniline, TDA, formaldehyde.The composition of the recycling stream can be monitored by on-line or off-line analysis for process control. Examples of suitable techniques include IR, Raman, NIR and UV-Vis spectroscopies or LC, HPLC, GC, GC-MS or NMR techniques, where these abbreviations have their well-known conventional meanings.

After removal of unreacted aniline and TDA, the polyamine mixture may be used directly for example as the curing agent for epoxy resins or may be hydrogenated to form the corresponding mixture of cycloaliphatic polyamines or may be processed further, for example by fractional distillation or fractional crystallisation, to produce more than one stream of different polyamine composition for example for production of the corresponding polyisocyanates.

After removal of unreacted aniline and TDA, the polyaromatic polyamine mixture can be converted to the corresponding polyisocyanates by reaction with phosgene in any of the methods described in the prior art. The derived polyisocyanate compositions are thus available for further use for a wide variety of applications, including use without further modification as the isocyanate component of polyurethanes or other applications, as the isocyanate component of prepolymers (by reaction with, for example, di- or polyfunctional polyether or polyester polyols or other materials with isocyanate-reactive functional groups) or variants (by formation of uretonimine, isocyanurate or other functional groups) and for further processing.

This further processing such as by means of fractional distillation and/or fractional crystallisation can be used to separate some or all of the lower molecular weight components in the polyisocyanate mixture to produce a polyisocyanate mixture with a different composition. The separated lower molecular weight polyisocyanates may themselves be used without further modification as the isocyanate component of polyurethanes or other applications, as the isocyanate component of prepolymers (by reaction with, for example, di- or polyfunctional polyether or polyester polyols or other materials with isocyanate-reactive functional groups) or variants (by formation of uretonimine, isocyanurate or other functional groups) and for further processing.

This further processing such as by means of further fractional distillation and/or further fractional crystallisation can be used to produce other compositions such as, for example, relatively pure 4,4'-MDI; mixtures of predominantly 4,4'-MDI and 2,4'-MDI; mixtures of MDI and MTI isomers in various proportions; mixtures of predominantly MTI isomers; or relatively pure individual MTI isomers.

The present invention is explained by means of the following examples, but the present invention is not limited to or by these examples.

### EXAMPLE 1

Toluene diamine (the "80/20" mixture of 2,4- and 2,6-TDA isomers) was preheated in an oven at 95 °C . In a beaker, 75.4g (0.81 mol.) aniline was weighed, then heated on a stirrer/hotplate and 75.0 g (0.61 mol.) of the liquid TDA was added, mixed, transferred to a sample bottle and stored in an oven at 65 °C.

In a nitrogen current, 466.0 g (4.98 mol.) of aniline was added to a 1-liter pressure-reactor, agitation was started and the aniline was temperature conditioned at 20 °C. Then 152,8 g (1.27 mol.) of 30.4 % hydrochloric acid was added drop-wise in 14 minutes, such that the temperature increased up to 41 °C. The solution was conditioned for 10 minutes at 40 °C. Next, 146.8 g (2.30 mol.) of 47 % formaldehyde was pumped in to the stirred reactor during 180 minutes with a flow rate of 0.71 ml/min while maintaining the temperature at 40 °C.

Next the temperature was increased and after reaching 60 °C, 141.1 g of the Toluene diamine/Aniline solution (respectively 0.58 and 0.76 moles) was added. The overall ratio of reactants used at this stage is thus An/TDA/F/HCl = 2.50/0.25/1.0/0.55

The reactor was closed, the temperature was increased to 90 °C and the reactor was pressurized up to 0.5 barg. The temperature was maintained for 30 minutes at 90 °C, followed by a temperature increase to 130 °C in 20 minutes (pressure to 1.5 barg), and maintaining the temperature for 90 minutes at 130 °C.

Next the temperature was decreased in 15 minutes to 80 °C and the solution collected and stored for convenience in an oven at 80 °C.

748 g of the reaction mixture was transferred to a 1-liter reactor and heated to 100 °C. 103 g (1.29 mol) of sodium hydroxide solution (50 wt%) was added and the mixture stirred for 5 minutes : separation of the layers was easily achieved in 5 minutes. The bottom layer (water phase) was discharged and 96 g. hot water was added to the remaining organic layer, stirred for 5 minutes and the phases allowed to separate for 5 minutes (temperature > 95 °C). The bottom layer (organic phase) was collected and the top layer (water phase) was discharged. The organic phase was rinsed for another 4 times with 100 g of hot water each time to yield 691 g of the raw amine mixture.

The composition of the raw amine as determined by gel permeation chromatography using refractive index detection was as follows:

| | |
|---|---|
| Aniline | : 28.1 wt% |
| Toluene diamine isomers | : 2.1 wt% |
| Diphenylmethanes | : 43.8 wt% |
| Triphenylmethanes | : 16.5 wt% |
| Polyamines (tetra + penta + etc) | : 9.4 wt% |

The diphenyl methanes are a mixture of MDA isomers and MTA isomers. The individual MDA isomers can be determined by gas chromatographic analysis using flame ionisation detection which gave the following results :-

| | |
|---|---|
| 4,4'-MDA : | 30.2 wt% |
| 2,4'-MDA : | 3.8 wt% |
| 2,2'-MDA : | 0.1 wt% |

The amount of MTA isomers can be estimated by difference as 9.7 wt% [43.8-30.2-3.8-0.1]. The presence of minor amounts of diphenylmethane impurity species such as N-methylated variations of the main compounds [e.g. H₂N-Ph-CH₂-Ph-NH-CH₃] are acknowledged to give some inaccuracies in the determination of the MTA concentration, but these will not detract significantly from the calculated abundance.

After removal of the bulk of the unreacted mononuclear amines by distillation, the total amount of N-methyl groups present in the polyamine mixture was measured by ¹H-NMR of a solution of the polyamine in deuterated chlorobenzene, after removal of labile H-atoms by exchange with D₂O. The ratio of N-methyl groups compared to methylene groups between aromatic rings was found to be 0.2 to 99.8 (no unreacted amino-benzyl-aniline species were detected : -CH₂-N- peaks not observed).

### EXAMPLE 2

Toluene diamine (the "80/20" mixture of 2,4- and 2,6-TDA isomers) was preheated in an oven at 95 °C. In a beaker, 75.4 g (0.81 mol.) aniline was weighed, heated on stirrer/hotplate and 150.2 g (1.23 mol.) of liquid TDA was added, mixed, transferred to a sample bottle and stored in an oven at 65 °C.

In a nitrogen current, 466.3 g (4.98 mol.) of Aniline was added to a 1-liter pressure-reactor, agitation started and conditioned at 20 °C, then 152,8 g (1.27 mol.) of 31.3 % hydrochloric acid was added drop wise in 8 minutes; the temperature increased to 39 °C. The solution was conditioned for 10 minutes at 40 °C.

Next, 146.8 g (2.30 mol.) of 47 % formaldehyde was pumped in to the stirred reactor during 180 minutes with a flow rate of 0.71 ml/min while maintaining the temperature at 40 °C.

Next the temperature was increased to 60 °C when 214.7 g of the Toluene diamine/Aniline solution (respectively 1.17 and 0.77 moles) was added. The overall ratio of reactants used at this stage is thus An/TDA/F/HCl = 2.50/0.5/1.0/0.57

The reactor was closed, the temperature was increased to 90 °C and the reactor was pressurized up to 0.5 bars. The temperature was maintained for 30 minutes at 90 °C, followed by a temperature increase to 130 °C in 20 minutes (pressure to 1.5 barg), and maintaining the temperature for 90 minutes at 130 °C.

Next the temperature was decreased in 15 minutes to 80 °C and the solution collected and stored for convenience in an oven at 80 °C.

805g of the reaction mixture was neutralised and worked-up in the same way as in the previous example to yield 798 g raw amine.

The composition of the raw amine as determined by gel permeation chromatography using refractive index detection was as follows:

| | |
|---|---|
| Aniline | : 30.9 wt% |
| Toluene diamine isomers | : 2.4 wt% |
| Diphenylmethanes | : 41.5 wt% |
| Triphenylmethanes | : 17.3 wt% |
| Polyamines (tetra + penta + etc) | : 7.8 wt% |

The diphenyl methanes are a mixture of MDA isomers and MTA isomers. The individual MDA isomers can be determined by gas chromatographic analysis using flame ionisation detection which gave the following results :-

| | |
|---|---|
| 4,4'-MDA | : 16.5 wt% |
| 2,4'-MDA | : 2.4 wt% |
| 2,2'-MDA | : 0.1 wt% |

The amount of MTA isomers can be estimated by difference as 22.5 wt% [41.5-16.5-2.4-0.1]. The presence of minor amounts of diphenylmethane impurity species such as N-methylated variations of the main compounds [e.g. H₂N-Ph-CH₂-Ph-NH-CH₃] are acknowledged to give some inaccuracies in the determination of the MTA concentration, but these will not detract significantly from the calculated abundance.

### EXAMPLE 3

After removal of unreacted aniline and TDA by distillation, the polyamine mixture obtained from Example 2 was subjected to phosgenation using the following procedure.

The polyamine mixture was heated in oven at 95 °C and 500 g monochlorobenzene was warmed in another oven at 45 °C.

In a beaker, 475 g of the warm monochlorobenzene was weighed, kept warm on a stirrer/hotplate and 25.0 g of the hot polyamine mixture added, mixed, and after all the polyamine had dissolved, transferred to a sample bottle and stored in an oven at 45 °C.

In a nitrogen current, 500 g of monochlorobenzene was added to a reaction vessel and cooled to 5 °C. 96 g of phosgene was added in 17 minutes, the mixture was cooled to 10 °C and the polyamine/monchlorobenzene solution added drop-wise while stirring during an addition time of 8 minutes; the temperature increased to 28.0 °C.

The mixture was slowly heated up to 110 °C to obtain a transparent liquid.

Nitrogen was purged through the solution to remove excess Phosgene. The excess monochlorobenzene was removed by using a Rotavap to obtain 25 g of a polyisocyanate mixture.

The dynamic viscosity of the polyisocyanate product was 338 cP and the NCO value was 35.7%. The relative amounts of MDI, MTI and the tri-phenylmethane versions (denoted below as 3-MDI and 3-MTI) of the polyisocyanate mixture were determined by gas chromatography using flame ionisation detection and were as follows (quoted relative to the total of the 3-MDI tri-isocyanate isomers) :

| | |
|---|---|
| Di-isocyanate (MDI) isomers | : 7.8 |
| MTI isomers | : 6.8 |
| 3-MDI isomers | : 1.0 |
| 3-MTI isomers | : 2.6 |

### EXAMPLE 4

Toluene diamine (the "80/20" mixture of 2,4- and 2,6-TDA isomers) was preheated in an oven at 95 °C.

In a beaker, 75.4g (0.81 mol.) aniline was weighed, then heated on a stirrer/hotplate and 75.2 g (0.62 mol.) of the liquid TDA was added, mixed, transferred to a sample bottle and stored in an oven at 65 °C.

In a nitrogen current, 468.0 g (5.0 mol.) of aniline was added to a 1-liter pressure-reactor, agitation was started and the aniline was temperature conditioned at 20 °C. Then 25 g (0.21 mol.) of 31.3 % hydrochloric acid was added drop-wise in 1.5 minutes, such that the temperature increased up to 26 °C. The solution was conditioned for 10 minutes at 40 °C.

Next, 146.8 g (2.30 mol.) of 47 % formaldehyde was pumped in to the stirred reactor during 178 minutes with a flow rate of 0.71 ml/min while maintaining the temperature at 40 °C.

Next the temperature was increased and after reaching 60 °C, 144 g of the Toluene diamine/Aniline solution (respectively 0.59 and 0.77 moles) was added. The overall ratio of reactants used at this stage is thus An/TDA/F/HCl = 2.50/0.26/1.0/0.09

The reactor was closed, the temperature was increased to 90 °C and the reactor was pressurized up to 0.5 bar. The temperature was maintained for 30 minutes at 90 °C, followed by a temperature increase to 130 °C in 20 minutes, and maintaining the temperature for 90 minutes at 130 °C.

Next the temperature was decreased in 15 minutes to 80 °C and the solution collected and stored for convenience in an oven at 80 °C.

634 g of the reaction mixture was transferred to a 1-liter reactor and heated to 100 °C. 18 g (0.23 mol) of sodium hydroxide solution (50 wt%) was added and the mixture stirred for 5 minutes : separation of the layers was easily achieved in 5 minutes.

The bottom layer (organic phase) was collected and the top layer (aqueous pahse) was discharged.

The organic phase was returned to the reactor and 122 g. hot water was added, stirred for 5 minutes and the phases allowed to separate for 5 minutes. The bottom layer (organic phase) was collected and the top layer (water phase) was discharged. The organic phase was rinsed for another 4 times with 100 g of hot water each time to yield 544 g of the raw amine mixture.

The composition of the raw amine as determined by gel permeation chromatography using refractive index detection was as follows:

| | |
|---|---|
| Aniline | : 30.8 wt% |
| Toluene diamine isomers | : 0.7 wt% |
| Diphenylmethanes | : 35.2 wt% |
| Triphenylmethanes | : 20.3 wt% |
| Polyamines (tetra + penta + etc) | : 13.1 wt% |

The diphenyl methanes are a mixture of MDA isomers and MTA isomers. The individual MDA isomers can be determined by gas chromatographic analysis using flame ionisation detection which gave the following results :-

| | |
|---|---|
| 4,4'-MDA | : 21.0 wt% |
| 2,4'-MDA | : 4.1 wt% |
| 2,2'-MDA | : 0.3 wt% |

The amount of MTA isomers can be estimated by difference as 9.8 wt% [35.2-21.0-4.1- 0.3]. The presence of minor amounts of diphenylmethane impurity species such as N- methylated variations of the main compounds [e.g. H₂N-Ph-CH₂-Ph-NH-CH₃] are acknowledged to give some inaccuracies in the determination of the MTA concentration, but these will not detract significantly from the calculated abundance.

After removal of the bulk of the unreacted mononuclear amines by distillation, the total amount of N-methyl groups present in the polyamine mixture was measured by ¹H-NMR of a solution of the polyamine in deuterated chlorobenzene, after removal of labile H-atoms by exchange with D₂O. The ratio of N-methyl groups compared to methylene groups between aromatic rings was found to be 0.1 to 99.9 (no unreacted amino-benzyl-aniline species were detected : -CH₂-N- peaks not observed).

## Claims

1. Process for manufacturing aromatic polyamine mixtures comprising the step of reacting formaldehyde with at least one mono-nuclear aromatic monoamine and one ore more aromatic di- or higher functionality amines.

2. Process according to claim 1 wherein the mononuclear aromatic monoamine is used as the main aromatic amine.

3. Process according to claim 1 or 2 wherein the mono-nuclear aromatic monoamine comprises aniline.

4. Process according to claim 3 wherein the amount of aniline is in the range 1.5 to 4.0 moles per mole of formadehyde.

5. Process according to any one of the preceding claims wherein the aromatic di- or higher functionality amines comprises one or more of the isomers of tolulene diamine.

6. Process according to claim 5 wherein a mixture of about 80 wt% 2,4'-tolulene diamine and about 20 wt% 2,6'-tolulene diamine is used.

7. Process according to claim 5 or 6 wherein the amount of tolulene diamine is in the range 0.1 to 0.8 moles per mole of formaldehyde.

8. Process according to any one of the preceding claims wherein reaction is carried out in the presence of an acid catalyst.

9. Process according to claim 8 wherein the acid catalyst comprises hydrochloric acid.

10. Process according to claim 9 wherein the amount of hydrochloric acid is in the range 0.05 and 0.60 moles per mole of formaldehyde.

11. Aromatic polyamine mixtures obtainable by the process as defined in any one of the preceding claims.

12. Process for manufacturing polyaromatic polyisocyanate compositions comprising the step of phosgenating the aromatic polyamine mixtures as defined in claim 11.

13. Polyaromatic polyisocyanate compositions obtainable by the process as defined in claim 12.
